# EUROPEAN PATENT APPLICATION

(11) **EP 4 424 228 A1**
(43) Date of publication of application: **04.09.2024**
(21) Application number: 24160100.4
(22) Date of filing: 27.02.2024
(51) Int. Cl.: A61B 1/005, A61B 1/00

(54) **ENDOSCOPE HANDLE STEERING CONTROLS**

(30) Priority: 03.03.2023 US 202363488220 P; 28.06.2023 US 202363510781 P
(71) Applicant: Cook Medical Technologies LLC, Bloomington, IN 47404 (US)
(72) Inventor: CARRUTHERS, Christopher A., Winston-Salem, 27104 (US); SIGMON, JR. John C., Winston-Salem, 27104 (US); GITTARD, Shaun D, Winston Salem, 27104 (US); LUPTON, Jonathan K., Thomasville, 27360 (US); BRECHT, Michael, Pfafftown, 27040 (US); BREEN, Liam, Ballina, V94 E4CA (IE)
(74) Representative: Leach, Sean Adam

(57) **Abstract**

A medical scope device may have a first steerable device extending to a distal end of the medical scope device. The medical scope device may also include a second steerable device extending to the distal end of the medical scope device. The device may also include a first steering actuator. A selector switch may be mechanically coupled to the first steering actuator, where the first steering actuator controls a movement of a distal end of the first steerable device when the selector switch is in a first position, and where the first steering actuator controls a movement of a distal end of the second steerable device when the selector switch is in a second position.

## Description

### CROSS-REFERENCE TO RELATED APPLICATIONS

This application is a non-provisional application which claims priority to U.S. provisional application Serial Nos. 63/488,220, filed March 3, 2023, and 63/510,781, filed June 28, 2023, each of which is incorporated by reference herein in its entirety.

### TECHNICAL FIELD

Embodiments disclosed herein generally relate to medical endoscopes. More particularly, these embodiments relate to user-operable control systems for utilizing an endoscope device.

### BACKGROUND

The statements in this section merely provide background information related to the present disclosure and may not constitute prior art.

The traditional endoscope is a medical device used in a variety of procedures. For example, a physician may insert the endoscope, for example, into a patient's mouth or into another body opening and then manipulate the distal end of the device through the patient's gastrointestinal (GI) tract to perform a particular endoscopic procedure. The physician may then use a variety of instruments during the procedure that are passed through an accessory channel that is located within the outer shaft of the endoscope. As the endoscopy field advances, new endoscopes are being created for specific procedures.

Endoscopes are typically steerable from a proximal-end handle. For example, certain steering features and mechanisms for controlling the distal end of the endoscope are discussed in U.S. Patent Application Pub. No. 2015/0366435, which is hereby incorporated by reference in its entirety. While steerable endoscopes are used with success to treat a variety of issues, existing endoscopes are often challenging to steer when inside the body, particularly when the outer shaft of the endoscope is steered separately from components extending through the accessory channel (e.g., meaning two separate steering systems are needed). As such, the present disclosure presents an improved steering system for use with a variety of endoscopes where a single steering system may control the steering of multiple components in a selective manner.

### BRIEF DESCRIPTION OF THE DRAWINGS

In order that the present disclosure may be well understood, there will now be described various forms thereof, given by way of example, reference being made to the accompanying drawings. The components in the figures are not necessarily to scale. Moreover, in the figures, like-referenced numerals designate corresponding parts throughout the different views.
FIG. 1 is an illustration showing a steerable medical device in accordance with certain aspects of the present disclosure.
FIG. 2 is an illustration showing an embodiment of a handle for use with a steerable medical device in accordance with certain aspects of the present disclosure.
FIG. 3 is an illustration showing a portion of a steering system that selectively controls two separate elongated steerable components in accordance with certain aspects of the present disclosure.
FIG. 4 is an illustration showing a portion of the steering system of FIG. 3, including two spline shafts and four wire drums in accordance with certain aspects of the present disclosure.
FIG. 5 is an illustration showing a portion of the steering system from FIGS. 3-4, including a selector rod for switching steering control from a first elongated steerable component to a second elongated steerable component in accordance with certain aspects of the present disclosure.
FIG. 6 is an illustration showing a portion of the steering system of FIGS. 3-5, further including a locking ring in accordance with certain aspects of the present disclosure.
FIG. 7 is an illustration showing a portion of the steering system of FIGS. 3-6 in a first state such that a first elongated steerable component is controlled by the steering system in accordance with certain aspects of the present disclosure.
FIG. 8 is an illustration showing the portion of the steering system of FIG. 7, where the steering system has moved into a second state such that a second elongated steerable component is controlled by the steering system in accordance with certain aspects of the present disclosure.
FIG. 9 is a set of illustrations (9a-9j) showing options for selectable friction engagement between a wire drum and another component in accordance with certain aspects of the present disclosure.
FIG. 10 is an illustration showing a set of control wheels, where at least one of the control wheels is engageable by a brake lever in accordance with certain aspects of the present disclosure.
FIG. 11 is an illustration showing a second view of certain features depicted in FIG. 10, including two control wheels and the first brake lever.
FIG. 12 is an illustration showing a brake lever for engaging at least one control wheel in accordance with certain aspects of the present disclosure.
FIG. 13 is an illustration showing a braking surface forming a wedge for coupling with the brake lever of FIG. 12 in accordance with certain aspects of the present disclosure.
FIG. 14 is an illustration showing a set of control wheels, where at least one of the control wheels is engageable by a brake dial in accordance with certain aspects of the present disclosure.
FIG. 15 is an illustration showing a second view of certain features depicted in FIG. 14, including two control wheels and the brake dial.
FIG. 16 is an illustration showing the brake dial from FIGS. 14-15 in isolation.
FIG. 17 is an illustration showing a control wheel with surface features that are configured for engagement with a brake dial in accordance with certain aspects of the present disclosure.
FIG. 18 is an illustration showing another embodiment of a steering system for steering at least two elongated steerable components in accordance with certain aspects of the present disclosure.
FIG. 19 is an illustration showing the embodiment of the steering system from FIG. 18 in a first state or controlling a first elongated steerable component in accordance with certain aspects of the present disclosure.
FIG. 20 is an illustration showing the embodiment of the steering system from FIGS. 18-19 in a second state such that the steering system controls a second elongated steerable component in accordance with certain aspects of the present disclosure.
FIG. 21 is an illustration showing an electromechanical diagram for operating certain embodiments with the use of electronically-controlled motors (or other electromechanical actuators), potentially based on information received from a feedback loop, in accordance with certain aspects of the present disclosure.
FIG. 22A is an illustration showing a perspective view of an embodiment of a steering system for selectively controlling two steerable components having a rotatable know in accordance with certain aspects of the present disclosure.
FIG. 22B is an illustration showing a knob from the embodiment of FIG. 22A in isolation.
FIG. 22C is an illustration showing a perspective view of an embodiment of a steering system similar to that of FIG. 22A for selectively controlling two steerable components, and additionally for selectively providing fluid functionalities to said steerable components via a fluid control system in accordance with certain aspects of the present disclosure.
FIG. 22D is an illustration showing perspective view of an internal-facing knob surface for one embodiment of a mechanical interface.
FIG. 23 is an illustration showing a front view of the steering system shown in FIG. 22C.
FIG. 24 is an illustration showing a portion of the fluid control system depicted in FIG. 22C, including a fluid control housing and receives three fluid control rods in accordance with certain aspects of the present disclosure.
FIG. 25 is an illustration showing a top view of a portion of the steering system shown in FIG. 22C.
FIG. 26 is an illustration of a portion of the steering system shown in FIG. 22C, including a two-part wheel system for operation of a wire drum in accordance with certain aspects of the present disclosure.
FIGS. 27-28 show a portion of the fluid control system from FIG. 22C and FIG. 24 in a first state and a second state, where the first state and the second state correspond with respective steering states for respectively steering a first steerable device and a second steerable device in accordance with certain aspects of the present disclosure.
FIGS. 29-30 are illustrations showing a wire drum and wire harness in accordance with certain aspects of the present disclosure.

### DETAILED DESCRIPTION

The following description is merely exemplary in nature and is not intended to limit the present disclosure, application, or uses. It should be understood that throughout the drawings, corresponding reference numerals indicate like or corresponding parts and features.

In adding reference denotations to elements of each drawing, although the same elements are displayed on a different drawing, it should be noted that the same elements have the same denotations. In addition, in describing one aspect of the present disclosure, if it is determined that a detailed description of related well-known configurations or functions blurs the gist of one aspect of the present disclosure, it will be omitted.

In the following discussion, the terms "proximal" and "distal" will be used to describe the opposing axial ends of the device, as well as the axial ends of various component features. The term "proximal" is used in its conventional sense to refer to the end of the device (or component) that is closest to the medical professional during use of the assembly. The term "distal" is used in its conventional sense to refer to the end of the device (or component) that is initially inserted into the patient, or that is closest to the patient during use. The term "longitudinal" will be used to refer to an axial direction that aligns with the proximal-distal axis of the device (or component), for example, when the device is not bent. The terms "radially" and "radial" will be used to refer to elements, surfaces, or assemblies relative to one another that may extend perpendicularly from a longitudinal axis. The terms "circumference," "circumferentially," and "circumferential" will be used to elements, surfaces, or assemblies relative to one another encircling or substantially encircling a longitudinal axis at a radius.

The uses of the terms "a" and "an" and "the" and similar references in the context of describing the present disclosure (especially in the context of the following claims) are to be construed to cover both the singular and the plural, unless otherwise indicated herein or clearly contradicted by context. The use of the term "plurality of' is defined by the Applicant in the broadest sense, superseding any other implied definitions or limitations hereinbefore or hereinafter unless expressly asserted by the Applicant to the contrary, to mean a quantity of more than one. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context.

As used herein, the terms "comprise(s)," "include(s)," "having," "has," "can," "contain(s)," and variants thereof, are intended to be open-ended transitional phrases, terms, or words that do not preclude the possibility of additional acts or structures. The present description also contemplates other examples "comprising," "consisting of," and "consisting essentially of' the elements presented herein, whether explicitly set forth or not.

In describing elements of the present disclosure, the terms 1st, 2nd, first, second, A, B, (a), (b), and the like, may be used herein. These terms are only used to distinguish one element from another element, but do not limit the corresponding elements, irrespective of the nature or order of the corresponding elements.

Unless otherwise defined, all terms used herein, including technical or scientific terms, have the same meanings as those generally understood by those skilled in the art to which the present disclosure pertains. Such terms as those defined in a generally used dictionary are to be interpreted as having meanings equal to the contextual meanings in the relevant field of art.

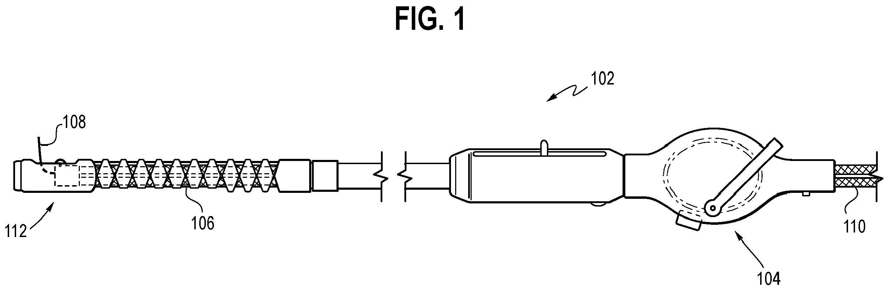

FIG. 1 shows an embodiment of a steerable medical device. The steerable medical device 102 includes a control handle 104 with a steerable outer sheath 106 extending distally therefrom. Without limitation, certain aspects of the steerable medical device 102 may include features discussed in U.S. Patent Application No. 16/749,083 (and corresponding Pub. No. 2020/0163534A1), which is hereby incorporated by reference in its entirety. For example, the steerable medical device 102 may generally include an endoscope (of any suitable type), and may include one or more accessory channels 110. The accessory channels may provide lumens or other pathways to a distal end 112 of the device 102 such that accessory components may extend to the device's distal end for performance of desired functions. For example, and without limitation, the accessory channels 110 may receive a cholangioscope 108, which is a relatively small-diameter device that is best delivered to a target site via a larger catheter (e.g., the outer sheath 106 and/or an accessory channel).

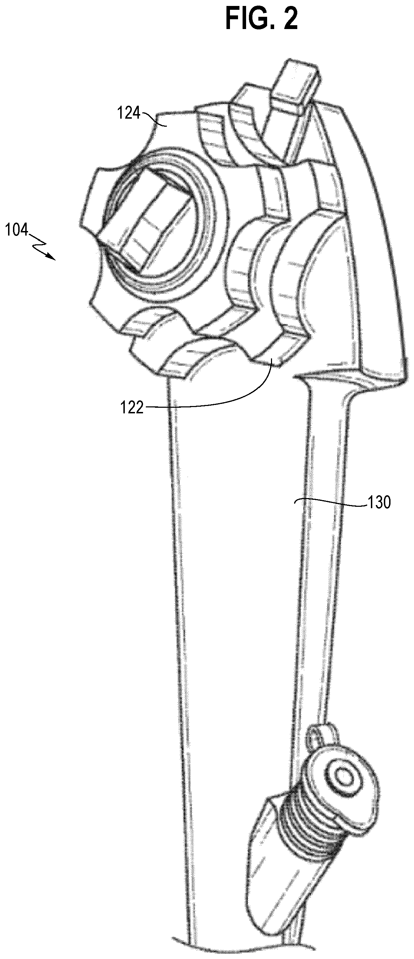

The handle 104 of the device 102 may be located at a proximal end of the steerable medical device 102, and therefore accessible directly by a medical professional. The handle may have any suitable features for steering the device, hereafter referred to as a steering control system 120. For example, an alternative handle 104 is shown in FIG. 2. Referring to FIG. 2, the steering control system 120 of the device includes a set of control wheels, in particular a first control wheel 122 and a second control wheel 124. The first control wheel 122 and the second control wheel 124 may be configured to control any suitable feature of the device, such as left-right and up-down motion of the outer sheath 106 of the steerable medical device 102. Certain aspects of the handle 104 and the control wheels may include features described U.S. Patent Application No. 15/655,239 (published as U.S. 2018/028778A1), entitled "STEERABLE MULTILUMEN CATHETER SHAFT," which is hereby incorporated by reference in its entirety.

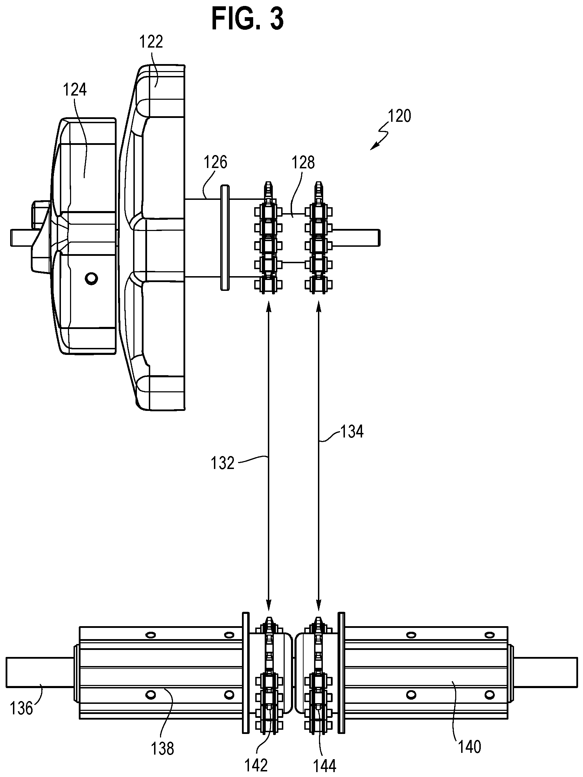

Referring to FIGS. 3-8, and in contrast to the prior art, the steering control system 120 may be configured for steering more than one component, and the particular component under control may be selectable/switchable at the demand of the user. More particularly, the first control wheel 122 and the second control wheel 124 may be configured to steer multiple elongated components rather than a single component. For example, in one non-limiting exemplary embodiment, the first control wheel 122 and the second control wheel 124 may be configured to control the outer sheath 106 (also referred to as an exoskeleton or the "first steerable device") of the medical device in one setting, e.g., a "first state," but may lack the ability to control the outer sheath 106 when the medical device is in a second setting, e.g., a "second state." When in the second state, the first control wheel 122 and the second control wheel 124 may instead steer or otherwise control a separate elongated device, such as a cholangioscope or other device (i.e., the "second steerable device") that extends through an accessory channel of the outer sheath 106. While the present disclosure generally describes the steering control system 120 as switching between the control of (1) the outer sheath 106, and (2) a cholangioscope, those skilled in the art will recognize that the steering aspects discussed herein may apply to any suitable endoscopy device where steering ability is desirable, and particularly those components that are elongated such that steering is desirable at or near the distal end of an endoscope. For example, any of the following may be controlled via aspects discussed herein: accessory channels, cameras, graspers, forceps, needles, brushes, stents, dissectors, suture holders, balloons, extraction baskets, lithotripsy probes, laser fibers, and/or any other suitable endoscopy or other medical device. Also, it is noted by the inventors that the two steerable devices may also be moveable distally and/or proximally relative to each other, for example when an inner device advances distally relative to an outer sheath. Steering of either device may occur when one steerable device is advanced distally relative to the other, for example.

Notably, the steering system 120 is depicted as having the first control wheel 122 and the second control wheel 124 as its only steering actuators. Other actuator types are also contemplated as an alternative (or addition) to the wheels, such as switches, sliders, levers, one or more electronic controls (utilizing a joystick, motor, etc.) and the like. In the present embodiment, the first control wheel 122 and the second control wheel 124 are coaxial, rotatable relative to each other (and each rotatable relative to a handle or housing of the medical device 102), and generally adjacent to one another to facilitate one-hand use. Advantageously, a physician or other medical professional may find this embodiment relatively easy to operate (perhaps with a single hand), particularly where the actuators are capable of controlling multiple steerable devices in a selectable manner (as discussed in more detail below).

As shown in FIG. 3, a first axle 126 may be fixed to the first control wheel 122 and a second axle 128 may be fixed to the second control wheel 124. Each of the first axle 126 and the second axle 128 may extend inside the handle 104 of the device such that they are generally hidden from view (e.g., see FIG. 2 showing where the handle housing 130 may be located relative to the wheels and axles). The second axle 128 may be hollow such that the first axle 126 may extend through the second axle 128 in a co-axial manner. Since the first axle 126 may need to be exposed within the handle of the device to reach a first strand 132 (discussed below), the first axle 126 may be longer than the second axle 128 such that an end of the first axle 126 within the handle of the device is exposed outside of the hollow second axle 128. Optionally, the axles may be supported by a central shaft 136 which is fixed relative to the handle's housing, and which may generally extend along the rotational axis of each of the first control wheel 122, the second control wheel 124, the first axle 126, and the second axle 128.

The first axle 126 and the second axle 128 may be mechanically coupled to respective spline shafts (or other suitable device for manipulating a pull wire of the steerable medical device 102, discussed below). For example, referring to FIG. 3, the first axle 126 may include a sprocket (or other suitable connection element) that couples to a first strand 132, and the second axle 128 may similarly include a sprocket (or other suitable connection element) that couples to a second strand 134. Herein, the term "strand" may include a chain with a plurality of individual links, but it also is defined to encompass belts, ropes, strings, and any other suitable elongated cord or strand for connecting offset rotatable elements. Optionally, a motor may be utilized (e.g., connecting a steering actuator, which may be a motor switch), and the spline shaft, but this is optional.

The first strand 132 may drive a first spline shaft 138, and the second strand 134 may drive a second spline shaft 140. Thus, when the first control wheel 122 is actuated by a user, mechanical action may cause the first spline shaft 138 to rotate, and when the second control wheel 124 is actuated by the user, mechanical action may cause the second spline shaft 140 to rotate. Optionally, the mechanical action may be associated mechanical advantage (i.e., via different diameters where the strands respectively engage the axles and spline shafts) such that a single rotation of a control wheel causes more or less than a single rotation of the respective spline shaft.

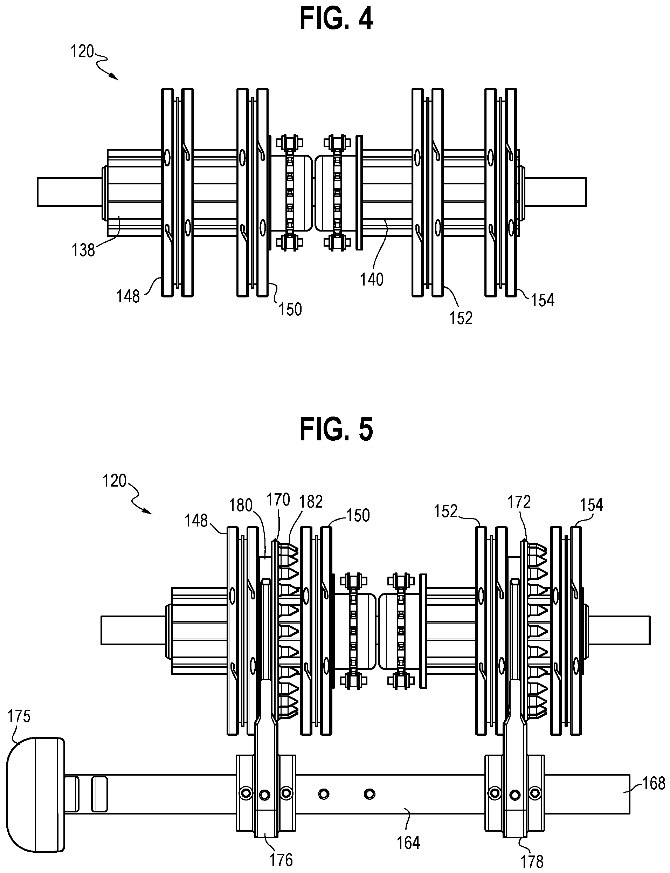

FIG. 4 shows particular features of the first spline shaft 138 and the second spline shaft 140. Optionally, the first spline shaft 138 and the second spline shaft 140 may be substantially the same in structure (e.g., mirrored as shown), and thus features discussed for one spline shaft may also be applicable to the other. For simplicity of description, certain features are discussed only with reference to the first spline shaft 138, but they shall be understood as also applicable to the second spline shaft 140.

Referring to the first spline shaft 138, a first sprocket 142 (or other strand-receiving feature) may be located at an end of the first spline shaft 138 for receiving the first strand 132 (discussed above). This strand may control rotation of the first spline shaft 138 about a central shaft 136, which may be fixed to the handle's housing and act as a support. While it is contemplated that the first spline shaft 138 may be slidable along the central shaft 136, certain non-limiting exemplary embodiments (including the depicted embodiment) have the first spline shaft 138 as moveable only via rotation (i.e., it is fixed from axial movement along the central shaft 136). Similarly, a second sprocket 144 may be located on the second spline shaft.

The first spline shaft 138 may be associated with at least one wire drum, such as a first wire drum 148 and a second wire drum 150 (as depicted). Each of the first wire drum 148 and the second wire drum 150 may include a wire groove that receives a pull wire, the pull wire extending to the distal end of the device for controlling certain distal-end movements. For example, the first wire drum 148 may manipulate a control wire extending to the first steerable device and the second wire drum 150 may manipulate a control wire extending to the second steerable device. In other embodiments, additional wire drums may be included (e.g., for additional steerable devices and/or for additional movement types).

In some embodiments, it may be advantageous to wrap a control wire around a wire drum such that it abuts about 180 degrees, or more, of the drum circumference, before anchoring it to the drum. This aspect prevents or substantially limits wire kinking when the drum rotates beyond 180 degrees (e.g., in a rotation direction that is opposite the direction the wire is initially wrapped around the drum). This aspect may be applicable to any of the above-described wire drums.

Similarly, the second spline shaft 140 may be associated with a third wire drum 152 and a fourth wire drum 154, with each of these wire drums being configured to manipulate a respective control wire. While any suitable arrangement is contemplated, the depicted embodiment has the third wire drum 152 controlling the first steerable device and the fourth wire drum 154 controlling the second steerable device. For example, the first wire drum 148 may control the position of the first steerable device in a first direction (optionally left-to-right), and the third wire drum 152 may control the position of the first steerable device in a second direction (optionally up-and-down). The second wire drum 150 and the fourth wire drum 154 may control the second steerable device in a similar manner. It may be particularly advantageous for all wire drums associated with the first spline shaft 138 to be associated with a first direction of movement, thereby creating a user-friendly control system having the first control wheel 122 associated with a single movement type, but this is not required. Similarly, the third wire drum 152 and fourth wire drum 154 may cause a similar movement between the first steerable device and the second steerable device.

Each wire drum may have an engaged state, where the wire drum is substantially fixed to its respective spline shaft (i.e., where rotation of the spline shaft causes corresponding rotation of the wire drum). Additionally, at least a portion of the wire drums (and perhaps all of them) may have an unengaged state where rotation of the respective spline shaft does *not* cause corresponding rotation of the wire drum. Referring to FIG. 3, a selector rod 164 is included, which may control which wire drums are engaged to the control wheels. While the inventors have found that a selector rod 164 is particularly advantageous for its reliability and ease-of-use, it is noted that other suitable devices for engaging/disengaging the wire drums may be included, such as switches, levers, locks, grips, or the like.

The depicted selector rod 164 generally includes shaft 168 that is coupled to a first collar 170 and a second collar 172. A knob 175 at the end of the shaft 168 may be accessible from outside the handle housing such that a user can directly move the knob, thus causing the collars to move. The first collar 170 may communicate with the first wire drum 148 and the second wire drum 150, and optionally may be located generally between these two drums in all operational states.

The first collar 170 may be located on a first collar mount 176, where the first collar mount 176 is fixed from movement relative to the selector rod 164. The first collar 170 may be generally fixed in linear directions relative to the first collar mount 176, but it may be rotatable relative to the first collar mount 176 about the central shaft 136. For example, the first collar mount 176 may be at least partially received by a collar groove 180 that prevents axial-direction movement of the first collar 170, but where such engagement allows rotation of the first collar mount 176. The second collar 172 may be located on a second collar mount 178.

An inner diameter area of the first collar 170 may include protrusions that extend within collar grooves of the first spline shaft 138. This engagement may fix the first collar 170 relative to the first spline shaft 138 in the context of rotation about the central shaft 136, meaning the first collar 170 will rotate when the first spline shaft rotates. At the same time, the first spline shaft 138 may be slidable along the first spline shaft 138 (e.g., where the above-mentioned extrusions slide within the collar grooves) such that the first collar 170 may switch engagement between the first wire drum 148 and the second wire drum 150.

The first collar 170 may include one or more collar teeth 182 that are configured to rotationally-fix the first collar 170 to a wire drum, such as either the first wire drum 148 or the second wire drum 150. When the collar teeth 182 engage a respective wire drum, they are designed to substantially prevent rotation of the wire drum relative to the first collar 170. Thus, when the first collar 170 rotates, the engaged wire drum must also rotate. As a result, and since the first collar 170 is fixed from rotating relative to the spline shaft, rotation of the first spline shaft 138 causes rotation of the wire drum that is coupled to the first collar 170.

While any suitable number of collar teeth are contemplated, the inventors have found the range of 4-60 teeth to be particularly suitable in certain exemplary embodiments, and more precisely from about 20 teeth to about 45 teeth. Notably, as the number of teeth is increased, an unexpected advantage is that a deflected steerable device (e.g., a cholangioscope) does not fall out of its deflected state (or only minimally so) as it is locked and steering is switched to another steerable device, given that there is an increase in the number of "stopping points" corresponding to the number of teeth. Thus, having at least 20, at least 30, at least 40, or more, teeth may be advantageous relative to other embodiments. Additionally, the shape of the teeth may be particularly selected such that the teeth are configured to smooth the transition from one steering device to another. For example, as shown in FIG. 5, each tooth is tapered, coming to a fine point at its terminal end. Advantageously, this feature ensures the teeth are able to more easily find the receiving groove of the receiving holes of the drums. The receiving holes of the drums may have a corresponding chamfer or taper (not shown) surrounding the tooth-receiving openings to give the pointed teeth a lead in. Advantageously, such features provide a smooth transition during switching and reduce any potential for friction.

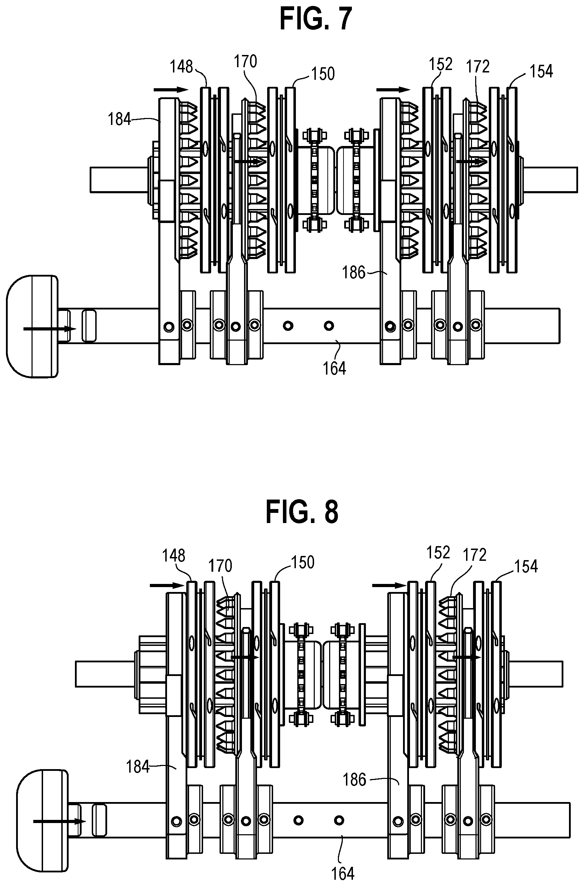

To illustrate an example of the device's operation, a "first state" is shown in FIG. 5 (and also in FIG. 7). In the first state, the selector rod 164 is in a position such that the first collar 170 engages the first wire drum 148 and the second collar 172 engages the third wire drum 152. Thus, the first wire drum 148 may be controlled via rotation of the first control wheel 122 and the second wire drum 150 may be controlled via rotation of the second control wheel 124. In this state, the second wire drum 150 is disengaged from the first spline shaft 138 such that it does not rotate when the first control wheel 122 rotates, and the fourth wire drum 154 is disengaged from the second spline shaft 140 such that it does not rotate when the second control wheel 124 rotates.

By contrast, in a "second state" (which is shown in FIG. 8), the selector rod 164 moves such that the first collar 170 engages the second wire drum 150 and the second collar 172 engages the fourth wire drum 154. As such, when the first spline shaft 138 rotates, the second wire drum 150 rotates, meaning rotation of the first control wheel 122 controls the second wire drum 150. Similarly, rotation of the second control wheel 124 causes rotation of the fourth wire drum 154.

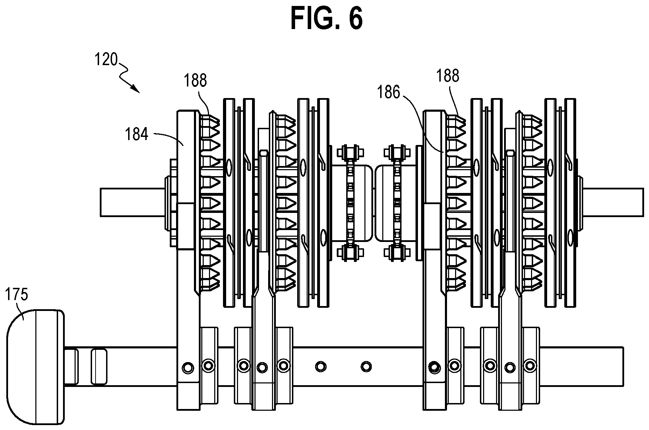

In some embodiments, optional features may be included that prevent the wire drums from rotating when they are *not* engaged to a respective collar. For example, referring to FIG. 6, a first lock ring 184 and a second lock ring 186 are included and fixed to the selector rod 164, meaning movement of the selector rod 164 causes movement of the lock rings. Lock rings may not be necessary in all embodiments, and notably they are not included in the embodiment of FIG. 5.

Referring to FIG. 6, the first lock ring 184 may have lock teeth 188 for engaging the first wire drum 148 (e.g., in a manner similar to the collar teeth 182 of the first collar 170). Similarly, the second lock ring 186 may have lock teeth 188 for engaging the third wire drum 152. When the selector rod 164 is in the "second state" discussed above (and as shown in FIG. 8), the first lock ring 184 may engage the first wire drum 148, preventing its rotation, and the second lock ring 186 may engage the third wire drum 152, preventing its rotation. Notably, the lock rings may substantially surround the spline shafts such that spline shaft rotation has no impact on the lock rings. In contrast to the first collar 170 and the second collar 172, the first lock ring 184 and the second lock ring 186 may lack rotatable components and may be substantially fixed (in all dimensions) to the selector rod 164, thereby ensuring substantial locking of an engaged wire drum when desirable.

In some embodiments, as an alternative to lock rings with lock teeth, the selector rod could have spring loaded pins that engage the drums when the steering is switched (and release when the steering is switched back to the opposite steering member). Any other suitable structure is also contemplated for coupling the wire drums for steering.

While not shown, those skilled in the art will appreciate that additional locking rings or other locking devices may be included. For example, the figures do not depict locking rings associated with the second wire drum 150 and the fourth wire drum 154, but such locking rings may be included.

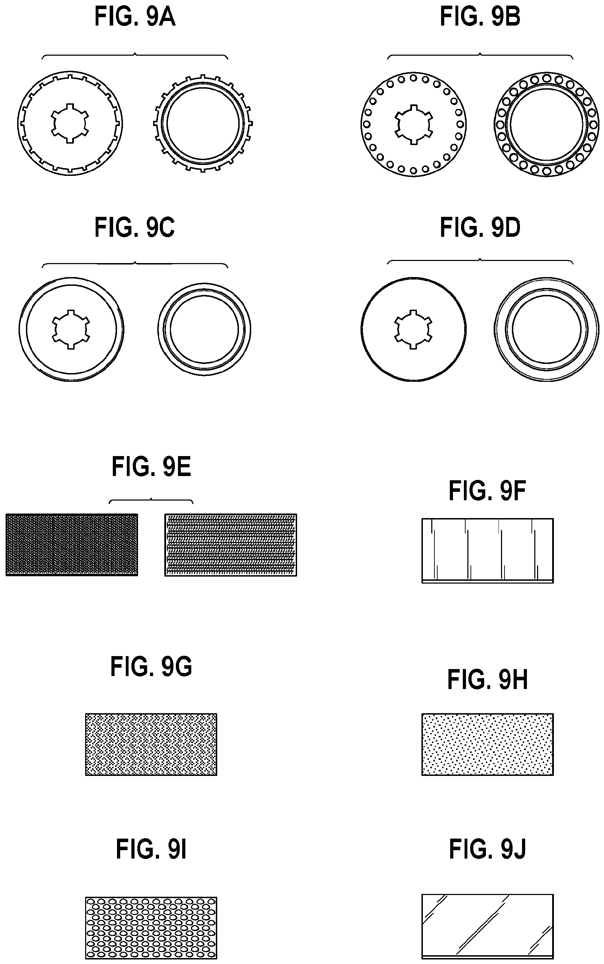

The wire drums may secure to associated components (e.g., the collar and/or locking ring discussed above) with any suitable securement structure capable of preventing rotation in a selective manner. In this paragraph, the associated component is referred to as the collar, but these features also apply to other components (e.g. the locking ring). In some embodiments, the engagement between the wire drum and the collar may engage via triangular synchromesh components shown in FIG. 9a. As shown in FIG. 9b, the assembly may include components that are securable via dogteeth. As shown in FIG. 9c, the assembly may include components that are securable via a wedge that causes suitable friction when in an engaged state. As shown in FIG. 9d, the components may have a simple friction design, which may be enhanced via the use of certain materials. For example (and without limitation), FIGS. 9e-9j show various friction materials that may be used with any of these designs (or as an alternative to these designs), including Velcro (FIG. 9e), a clutch pad (made of a rubber or other compliant material, FIG. 9f), a fine grit sandpaper (FIG. 9g), a course grit sandpaper (FIG. 9h), a sheet having a "bump" topography utilizing silicon (FIG. 9i), a flat sheet utilizing silicon with sufficient friction properties (FIG. 9j, which may be used as an alternative to "bump" topography), or the like. Countless other options are also contemplated.

In some embodiments, it is desirable to include brakes to prevent motion of the control wheels. Advantageously, when the control wheels are fixed from rotation in the embodiments of FIGS. 6-8, all wire drums in the depicted embodiments will also be prevented from rotating when the device is in the second state, as (1) the lock rings prevent the first wire drum 148 and third wire drum 152 from rotating, and (2) the locked spline shafts prevent the second wire drum 150 and the fourth wire drum 154 from moving. Thus, a single brake (or one for each control wheel) may brake both steerable components. This feature is enhanced if additional lock rings are included such that all wire drum rotation is prevented when the control wheels are braked regardless of whether the device is in the first state or second state.

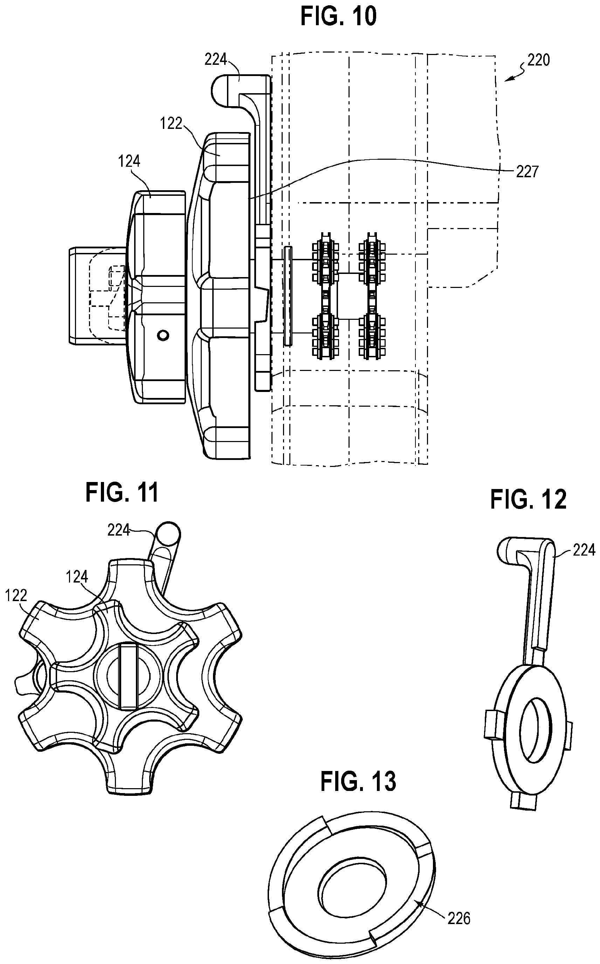

Any suitable brake may be included to prevent motion of the control wheels. For example, referring to FIGS. 10-13, a first brake 220 may be included, which may engage the first control wheel 122 to prevent rotation of the first control wheel 122. The first brake 220 may have any suitable structure. For example, the brake may include a brake lever 224 that has an angled braking surface 226, where rotation of the brake lever 224 causes the angled braking surface 226 to act as a wedge against a corresponding brake surface 227 located between the first control wheel 122 and the brake lever 224. As the brake lever 224 rotates further in a braking direction, the "wedge" aspect of the braking surface may increase static friction such that the first control wheel 122 cannot rotate, absent abnormal forces.

Additionally or alternatively, one or more of the brakes may be capable of providing variable resistance controlled by the physician, and in some embodiments a selected resistance level may be set and maintained by the device. For example, springs may be present such that once the brake position is determined for variable resistance, the spring resistance keeps the brake in such position even as the physician focuses on turning the steering controls (e.g., without worrying about also maintaining brake pressure). Other similar structures are also contemplated for adjusting and controlling brake force.

Optionally, a second first brake 222 may be included, which may function to prevent rotation of the second control wheel 124. Advantageously, having separate brakes may allow the medical professional to selectively brake in desired directions, allowing motion in only a single direction in certain circumstances. The second brake 222 may include a brake dial 228, which may rotate coaxially with the first control wheel 122 and the second control wheel 124. The brake dial 228 may include one or more angled surfaces 229 that abut or otherwise engage a corresponding angled surface 231 located on, or adjacent to, the second control wheel 124. Thus, when the brake dial 228 rotates, it may axially move away from the second control wheel 124 such that it pulls a braking surface 223 into engagement with the second control wheel 124, fixing it from rotation. E.g., in this non-limiting example, the braking surface 223 may be located on a rod that is rigidly coupled with the brake dial 228, where the rod enters into the handle and passes axially through the control wheels and terminates onto a braking pad that is inside the handle's shell. When the brake dial 227 is rotated, this rod may pull the brake into engagement with the second axle 128 (which is fixed to the second control wheel 124), thereby creating a friction brake preventing movement of the second control wheel 124.

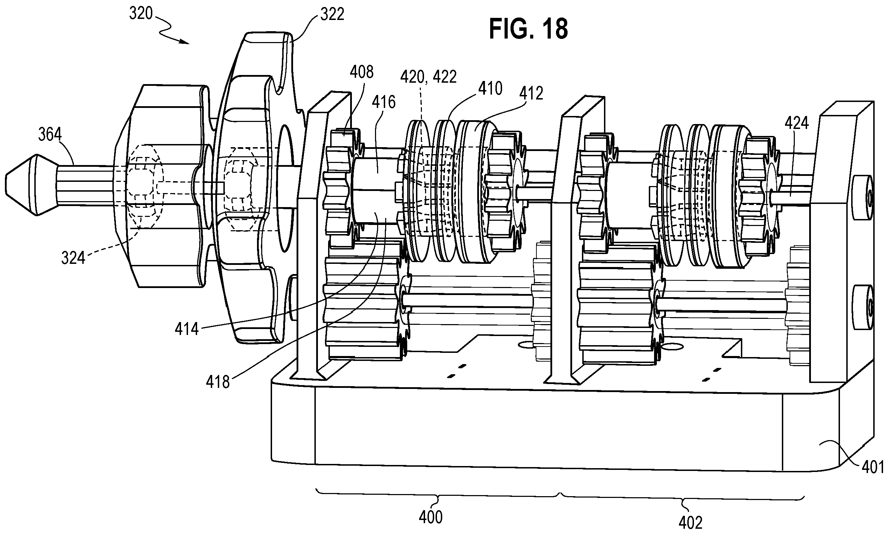

FIGS. 18-20 show another embodiment of a steering system 320, which has different mechanical features performing similar functions to the embodiments discussed above. A primary difference between this embodiment and those discussed above is that a selector rod 364 is coaxial with a first control wheel 322 and a second control wheel 324. Like the embodiments above, the selector rod 364 is configured to switch steering between a first steerable component to a second steerable component when it is moved axially.

A first portion 400 of the device, which is associated with the first control wheel 322, generally includes the first gearwheel 408, a first wire drum 410, and a second wire drum 412 (where the first and second wire drums control a particular motion of two respective steerable components, similar to as discussed above). The first portion 400 also includes a first collar 414 that is fixed to the first control wheel 322 such that when the first control wheel 322 rotates, the first collar 414 also rotates. The first collar 414 has two primary elements: (1) a first gearwheel 408 and (2) a first collar shaft 416, which may be formed integrally. The first collar shaft 416 has two portions along its length: a portion 418 without teeth and a separate portion 420 with outer diameter teeth 422.

When the steering system 320 is in the first state (for steering a first component), the first wire drum 410 may be engaged and the second wire drum 412 may be disengaged. The engagement of the first wire drum 410 may be accomplished via the outer diameter teeth 422 of the first collar shaft 416, which may engage corresponding teeth or grooves located on an inner diameter surface of the first wire drum 410. Due to this engagement, rotation of the first control wheel 322 causes rotation of the first wire drum 410. By contrast, the second wire drum 412 may be disengaged from the first control wheel 322 since it is generally held in place by a central shaft 424 that is coaxial with, but freely rotatable relative to, the first collar 414.

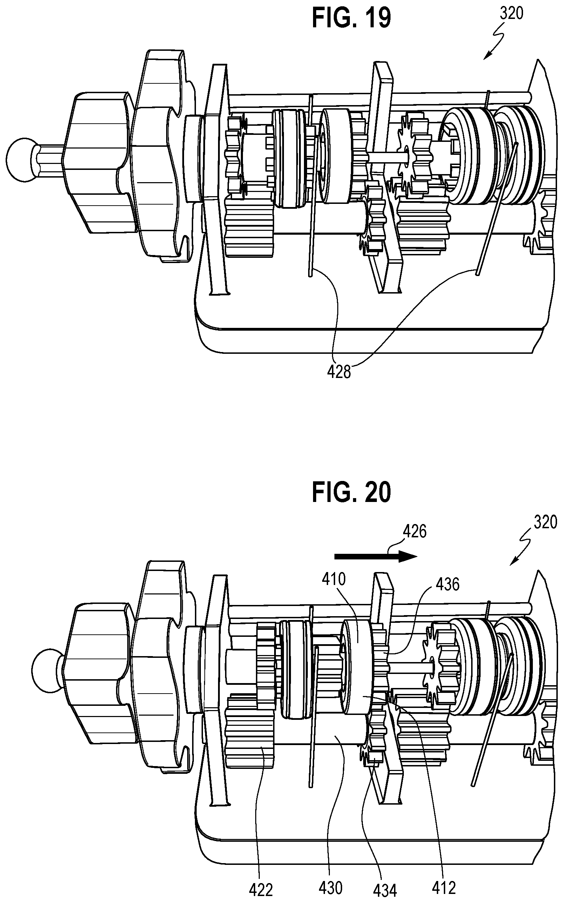

To adjust the device from the first state of FIG. 19 to the second state of FIG. 20, the selector rod 364 is moved in the direction identified by the arrow 426 shown in FIG. 20. This motion may cause several features to move linearly, including the first collar 414 and the second wire drum 412. Notably, this motion in particular causes the first collar 414 to move linearly relative to the first wire drum 410 (which may be substantially prevented from moving linearly due to the inclusion of a support arm 428 fixed to the handle body). This linear displacement between these components may cause the teeth 422 of the first collar 414 to slide out of engagement with the first wire drum 410, for example where the portion 418 without teeth now is aligned with the first wire drum 410. Thus, in this state, rotation of the first collar 414 has no impact on the first wire drum 410.

The first portion 400 of the steering system 320 may include a first countershaft 430 (labeled in FIG. 20 due to figure space constraints), which may function primarily to transfer rotation to the second wire drum 412 when the device is in the second state. The first countershaft 430 may include a first countershaft gearwheel 432 and a second countershaft gearwheel 434 that are rotationally fixed. The first countershaft gearwheel 432 may be engaged with the first gearwheel 408 of the first collar 414 such that rotation from the first control wheel 322 causes the first countershaft 430 to rotate.

In the second state of FIG. 20, the second wire drum 412 moves linearly such that it engages the second countershaft gearwheel 434, perhaps via teeth 436 that are fixed to the second wire drum 412. As a result, rotation of the first control wheel 322 causes the second wire drum 412 to also rotate, as the control wheel's rotation transfers from the first collar 414, to the first countershaft 430, and ultimately to the second wire drum 412. Notably, when the second wire drum 412 is moved into its position shown in FIG. 19 (the first state), its teeth 436 are not aligned with the second countershaft gearwheel 434 and therefore rotation of the first control wheel 322 has no impact on the second wire drum 412.

Similarly, a second portion 402 of the device includes features that generally correspond with those of the first portion 400, but that are instead rotated by the second control wheel 324. In other words, the second portion depicted in FIGS. 18-20 acts in a nearly-identical manner to the first portion (but controlled by a different wheel). As such, those with ordinary skill in the art will understand how the second portion 402 may operate in view of the description above with reference to the first portion 400. The first portion and the second portion may generally be fixed inside the handle via a housing 401, which may be secured to (or part of/integral with) the exterior handle housing.

Optionally, locking components may be included in the first portion 400 and/or the second portion 402, which may act similarly to the locking rings discussed above with reference to FIGS. 6-8. However, such components are not depicted in FIGS. 18-20 and are not necessary in all embodiments. Also, those skilled in the art will appreciate that certain aspects may be used that allow the central shaft 424 to cause linear movement (e.g., of the second and fourth wire drums) without necessarily causing rotational movement, which may be accomplished with a pin-and-groove connection or the like.

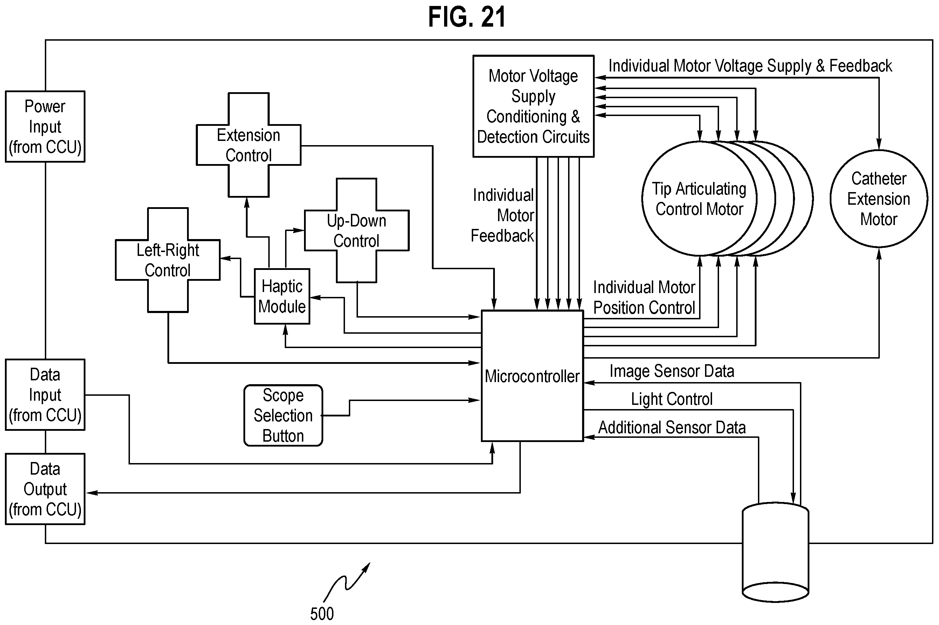

The aspects above may be controlled manually, where a human may directly control the knobs, fork selectors, and other components discussed above such that electronic control components are unnecessary. Other embodiments may include electromechanical aspects to automate certain motions, which may enhance the accuracy and precision of certain motions and reduce instances of human error. FIG. 21 shows an example of an electromechanical control system 500 for carrying out certain functions of a steering system similar to embodiments discussed above. For example, the electromechanical control system 500 may include one or more motors, which may include rotational or linear motor, servos, or other electromechanical modules for articulating the endoscope tip with the use of tension wires, strings or similar flexible material extending the endoscope length (from proximal to distal). When a tip articulation motor is included (as depicted) for generally steering the outer sheath of the device, the tip articulation motor may be shared, or one or more additional motors may be used for cholangioscope catheter, or another accessory, length extension from the distal tip.

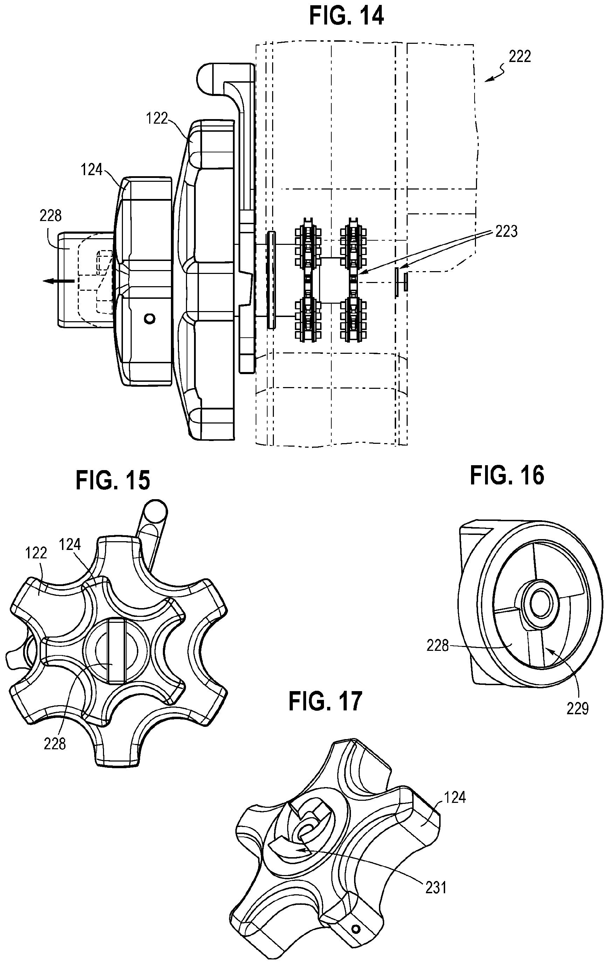

When included, the motors or other electronically-controlled actuators may be controlled by one or more microcontrollers located within the endoscope proximal handle assembly. Each motor (if there are multiple) may have an individual or common power supply, positional control, and/or motor status feedback (current, voltage, impedance, or other relevant electrical status). For example, using an encoder on the control wheels, which may be similar to those discussed above with reference to FIGS. 3-16, to control a corresponding servomotor or other type of motor that is coupled to the respective spline shaft. Thus, the controlled wheel is rotated and the motor rotates the spline shaft by a corresponding amount. The left/right brake and up/down brake (e.g., brakes as discussed above with reference to FIGS. 10-17) can then also be read by an encoder that then communicates to the motor to either stop or decrease degrees rotation per amount of rotation on the wheel. Advantageously, this motor power-control-feedback loop allows precise, accurate movement of each wire drum and can provide force, resistance, and/or tension feedback information for further processing, analysis, and use by the device microcontroller(s). The power-control feedback loop (with or without the microcontroller) may be embedded within each motor or contained within the proximal handle, along with an optional battery and/or other electronics. The motor(s) can be operated with or without the full power-control-feedback loop depending on component selection, desired level of control, and required status feedback for the rest of the system.

Position, via manipulation of the wire drums with the motors, may be controlled by turning the power on/off, switching power polarity, with a separate control signal, or a combination of these or any other suitable method. User control of motors may be provided via knobs, joysticks, buttons, or other user input devices located on the proximal handle assembly. A button, switch or other similar user input mechanism is provided for switching between the duodenoscope and cholangioscope. The user input device(s) can be either analog or digital with integrated electronics interpreting user input, raw electrical output such as variable resistors, or a combination of both or other similar implementations. Additionally, the user input device(s) and supporting electronics may be connected to the microcontroller(s), which may provide required filtering, interpretation, and transfer of user control input to motor control output.

Motor feedback described previously can also be used to provide haptic feedback to the user to simulate mechanical control and feedback via haptic modules included within the handle assembly. Haptic modules can include, but are not limited to, vibration, tension, force, torque. These haptic modules are controlled by the microcontroller(s) within the proximal handle assembly electronics, integrated within user input device(s) or a combination of both. Power is provided to the proximal handle assembly by a cable connection to the Camera Control Unit (CCU).

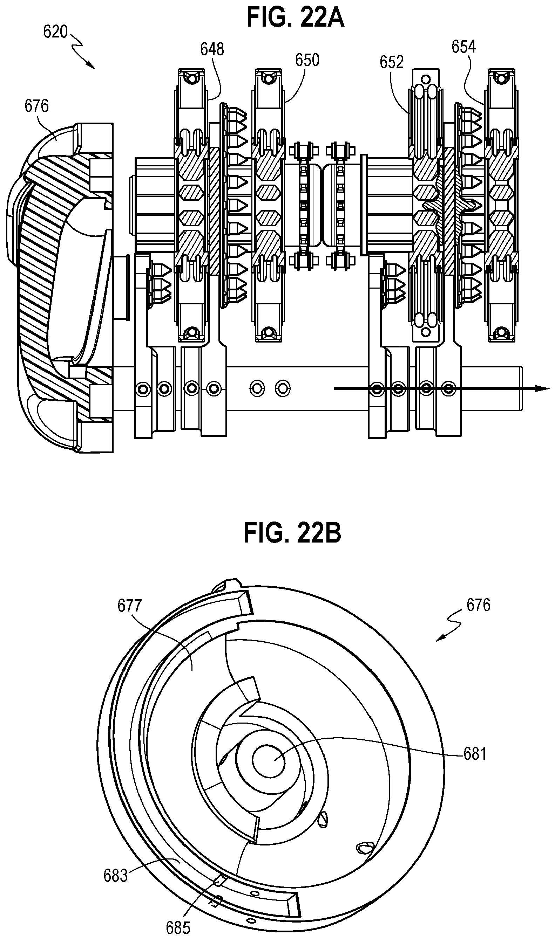

FIGS. 22A-C show an additional embodiment of a steering control system 620, which may incorporate any of the aspects discussed above, where suitable. Like in the embodiments discussed above, the selector rod 675 may be manually moveable to provide switchability of the steering control system 620. In the depicted embodiment, rather than utilizing a knob that is moveable via a linear sliding motion (as in certain embodiments above), the selector rod 675 is moveable via a knob 676 that is *rotatable.* This rotatable movement of the knob 676 may be translated to linear motion of the selector rod 675, for example. However, movement of the selector rod 675 may be accomplished via any other suitable control device or method (in this embodiment as well as others described herein). In some embodiments, for example, the selector rod 675 may be moveable via an electromechanical device, such as a motor that is actuated via the touch of a button and/or electronic command.

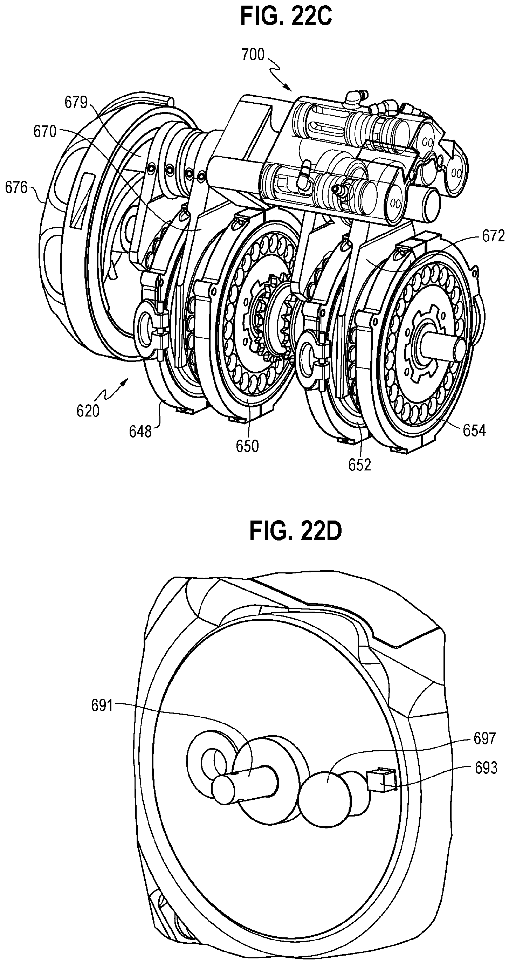

As shown in FIGS. 22A-D, the knob 676 may move the selector rod 675 mechanically. To accomplish this, the knob 676 may include a track 677 that receives a corresponding tab 679 fixed to the end of the selector rod 675 (where the tab 679 is shown in FIG. 22C). The track 677 may vary in depth such that it forms a portion of a spiral. Further, the knob 676 may remain linearly fixed relative to the housing of the device, perhaps via a pivot joint at a pivot opening 681. Thus, when the knob 676 turns/rotates, this rotational movement is translated into linear movement of the selector rod 675 relative to the device's housing. Optionally, the knob 676 may include a range groove 683 interacting with a corresponding pin 693 (shown in FIG. 22D) (or any similar feature) fixed to the housing that limits the total range of motion of the knob, such as to about 180 degrees as shown in the present embodiments. Other features of the housing (e.g., ball protrusion 697 and central protrusion 691) may correspond with the track 677 and the pivot opening 681, respectively. Advantageously, moving the knob to a terminal position (e.g., the limits of the range groove 683) may result in perfect (or near perfect) alignment of the selector rod 675 with a particular operational state, such as engagement with a particular wire drum. Additionally, a bump (e.g., bumps 685) or other feature may be present in the range grove to provide haptic feedback to the user that they are a operational state. Additional markings on the selector knob and handle shells may be present to provide visual confirmation to the user that they are in an operational state. The bump feature also prevents the knob from unintendedly shifting and leaving an operational state.

Any other suitable embodiment for moving the selector rod 675 is also contemplated. Optionally, when rotational motion is translated to linear motion (e.g., via the knob 676), an increased linear precision may be achieved via configuring the mechanical advantage characteristics such that a large amount of knob rotation causes only minimal linear motion, or vice versa.

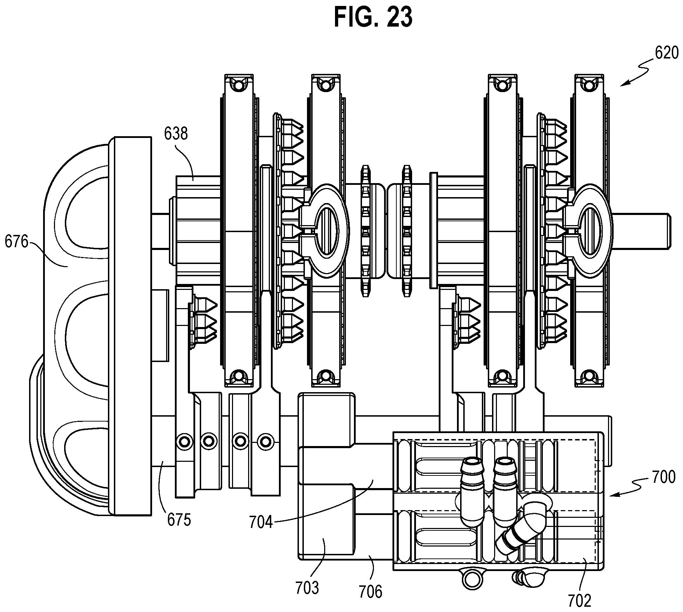

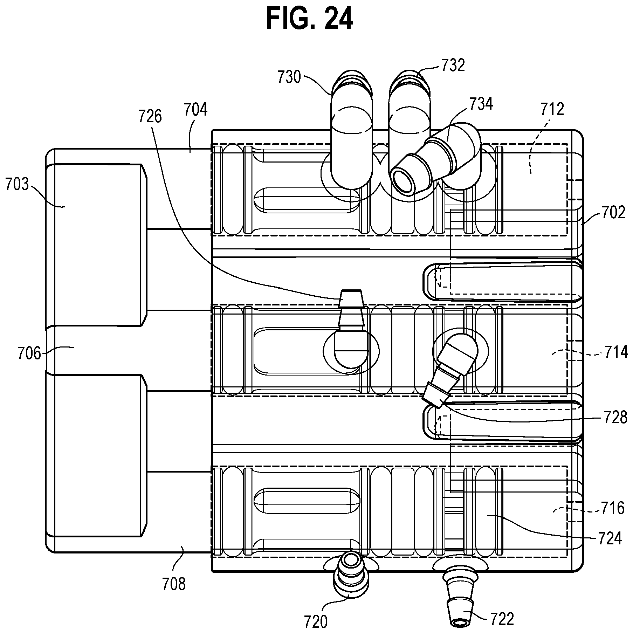

FIG. 22C, along with FIGS. 23-26 show the steering control system 620 having additional fluid-control features in addition to the steering aspects, where the steering aspects are similar to those of the steering control system 120 shown in FIGS. 1-16. Notably, the components and features of steering control system 120 are generally interchangeable/applicable with the steering control system 620, and vice versa. E.g., as depicted, the steering control system 620 includes four wire drums 648, 650, 652, and 654 that are selectively engageable by a first collar 670 and a second collar 672. Like the embodiments above, this arrangement provides the ability for a selector rod 675 to switch steering functionality between "a first steerable device" (e.g., an exoskeleton or other outer sheath of an endoscopic device, such as shown in FIG. 1) and "a second steerable device" (e.g., a cholangioscope or other suitable device, similar to as shown in FIG. 1).

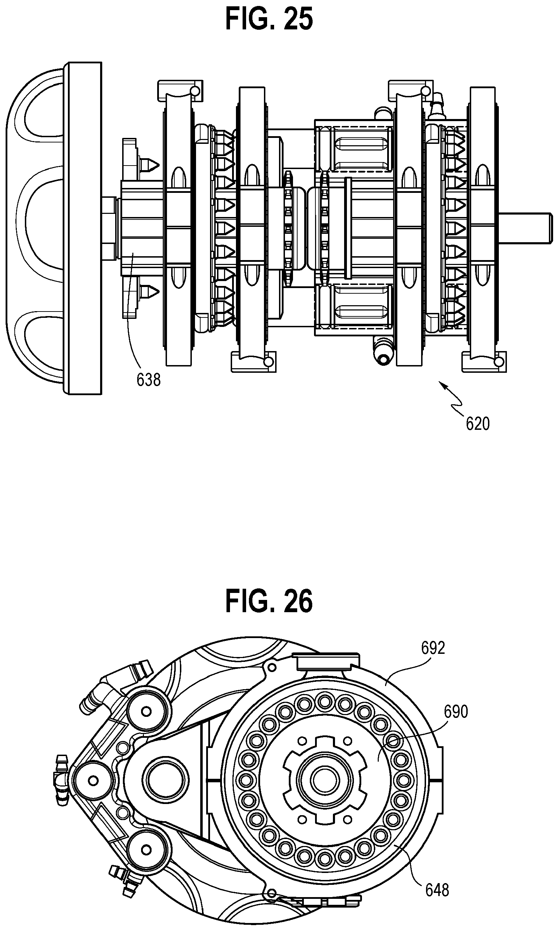

Referring to FIG. 26, the present embodiment utilizes two-part wheels for the wire drums. As depicted, the first wire drum 648 (which is representative of the others) is located on an outer perimeter surface of an inner wheel 690. The inner wheel 690 and the first wire drum 648 may be rotatable relative to each other, meaning the inner diameter surface of the first wire drum 648 may be slideable or otherwise moveable in a circumferential direction relative to an outer diameter surface of the inner wheel 690 such that the first wire drum 648 and the inner wheel 690 rotate about a common axis. All other degrees of freedom, such as 3D linear movement and/or rotation in another direction, may be prevented between the inner wheel 690 and the first wire drum 648. Both the wire drum 648 and the inner wheel 690 may be located inside a wheel housing or wire harness 692 to maintain the operational integrity and/or ensure guide wires remain engaged wire drum, though this feature is optional.

The inner wheel 690 may be generally fixed to the spline shaft 638 in all operational states. Thus, when the first wire drum 648 is not selected for operation (e.g., when the first wire drum 648 is not selected by the steering system's selector rod 675), rotation of the spline shaft 638 may cause rotation of the inner wheel 690 without causing the first wire drum 648 to rotation. By contrast, when the first wire drum 648 is selected for operation (e.g., selected by the steering system's selector rod), the first wire drum 648 and the inner wheel 690 may rotate together as the first spline shaft 638 rotates. Advantageously, by utilizing such a two-part wheel system to separate the wire drum(s) from the spline shaft(s), the inner wheel(s) and wire drums may be optimized for rotational freedom without manipulating the outer surface of the spline shaft(s).

In addition to switching the modes of steering, the selector rod 675 may also (or alternatively) be used to switch other functionalities between various devices. For example, as shown in FIGS. 22C-24, the steering control system 620 may include a fluid control system 700 that is capable of altering various fluid passageways such that various fluid-related functionalities can be turned on or off, and/or switched between certain sub-components of the medical device. For example, and without limitation, it is contemplated that suction, insufflation, and/or irrigation functionality at the distal end of the device may be affected by the position of the selector rod 675, as further described below.

The fluid control system 700 generally includes a fluid control housing 702 that receives one or more fluid control rods 704, 706, 708, which may be fixed to one another (and also the control rod 675) via a fluid control base 703. While "fluid control rods" are referred-to as "rods" herein, any other suitable control structure may be used. As shown in FIG. 23, the fluid control rods are fixed to the selector rod 675. That is, when the selector rod 675 moves, the fluid control rods 704, 706, 708 also move. Alternatively, the fluid control rods may be associated with a separate selector rod (to separate selectability of fluid functionality from steering), but this is not shown in the figures.

The fluid control housing 702 may remain generally fixed to a housing of the device such that movement of the fluid control rods 704, 706, 708 causes a first fluid control rod 704 to move within a first channel 712 of the fluid control housing 702, a second fluid control rod 706 to move within a second channel 714 of the fluid control housing 702, and a third fluid control rod 708 to move within a third channel 716 of the fluid control housing 702. While three rods (and three corresponding channels) are depicted, more or fewer than three may be included. Generally (and discussed further below), the fluid control rods are configured to control fluid communication amongst various ports, where "port" refers to a fluid inlet, outlet, or other interface to a fluid passage within the fluid control system.

In one non-limiting, example implementation, the first fluid control rod 704 is associated with an insufflation functionality of a first steerable device (e.g., an exoskeleton or other outer sheath). Insufflation, for example, may involve the introduction of a gas, such as air or carbon dioxide, into the area being examined. This helps to inflate the cavity, making it easier to see and examine the organs as an endoscopic steerable device is deployed. To achieve insufflation, when the steering control system 620 is in a first state (where the wire drums associated with a first steerable component are engaged for steering), the first fluid control rod 704 may be positioned to provide fluid communication between an insufflation inlet 720 and an insufflation outlet 722. This fluid communication may be provided by a cavity of the first fluid control rod 704, or other suitable feature, that aligns with each of the insufflation inlet 720 and the insufflation outlet 722, thereby allowing fluid to freely flow from the inlet to outlet (and vice versa). Notably (and optionally), the insufflation inlet 720 may still be pressurized and/or otherwise actuated when called by activation of an insufflation button located on an exterior portion of the device, meaning that fluid communication between the insufflation inlet 720 and insufflation outlet 722 may not immediately cause insufflation functionality (e.g., until an insufflation button is also pushed).

By contrast, when the first fluid control rod 704 is in a second state (where the wire drums associated with the first steerable component are disengaged), the first fluid control rod 704 may block fluid communication between the insufflation inlet 720 and the insufflation outlet 722. This prevention of fluid communication may be accomplished via directly blocking one or more of the insufflation inlet 720 and the insufflation outlet 722, or a path therebetween, with one or more seals 724 or other fluid-blocking structures. Advantageously, when steering of the first steerable device is turned "off," insufflation capabilities are also turned "off" this embodiment, which may be advantageous for ensuring a medical professional does not accidentally actuate insufflation when the first steerable component is not under the medical professional's control. Thus, even when an insufflation button or other actuator is pushed on an external portion of the device to activate insufflation of the first steerable component, such insufflation functionality does not and cannot occur at the distal end of the device due to lack of fluid communication between the insufflation inlet 720 and the insufflation outlet 722. O-rings or other sealing structures may be included to create and maintain a seal between the inlets and outlets in the appropriate settings (e.g., such as the seals 724).

The second fluid control rod 706 may be associated with a different functionality, such as an irrigation functionality of the first steerable device (e.g., where the first steerable device is capable of providing irrigation at its distal end when turned "on" and not capable of providing irrigation when "off"). Without limitation, in an irrigation functionality, the endoscope can be used to irrigate the area being examined with a sterile solution. This helps to clear away any debris or fluids that may be obstructing the view. Operation of the second fluid control rod 706 may be similar to that of the first fluid control rod 704, discussed above, e.g. where the second fluid control rod 706 controls fluid communication between an irrigation inlet 726 and an irrigation outlet 728.

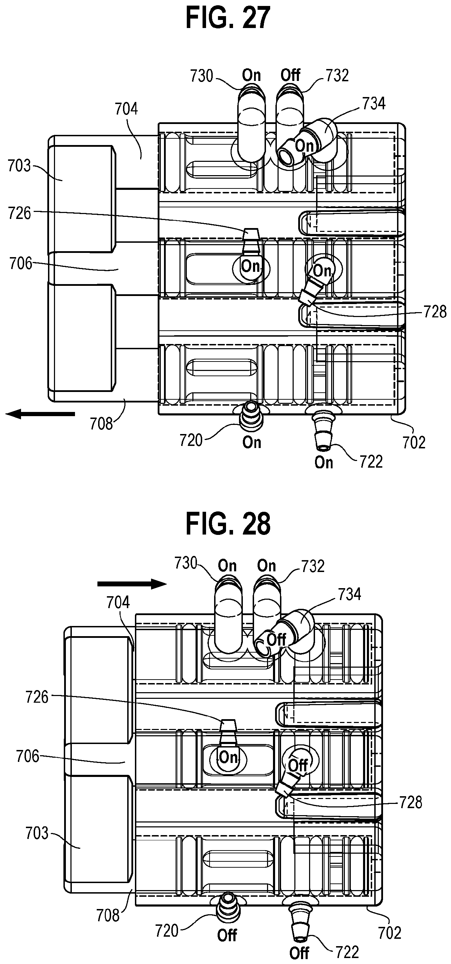

In addition to turning certain functionalities "off' and "on," the fluid control system 700 may also (or alternatively) be capable of switching a functionality between the first steerable device and the second steerable device. For example, the third fluid control rod 708 may be associated with a suction interface 730. In a first state (shown in FIGS. 23 and 27, where the first steerable device is engaged for steering), the third fluid control rod 708 may cause the suction interface 730 to be in fluid communication with a first suction port 732 that leads to the distal end of the first steerable device, meaning the first steerable device includes a suction functionality (which may be activated via an external button). In this state, a second suction port 734, which leads to the distal end of the second steerable device, is fluidly isolated from the suction interface 730, meaning the second steerable device does not have suction functionality in this state.

In a second state (shown in FIG. 28, where the second steerable device is engaged for steering such as in FIG. 8), the third fluid control rod 708 is positioned such that fluid communication is provided between the suction interface 730 and the second suction port 734. In this state, the second steerable device now includes suction functionality, while the first steerable device does not. In summary, by moving the selector rod 675 of the steering control system 620, steering control is changed from one steerable device to another, *and also* a fluid functionality (in this case suction) is also switched from the one steerable device to the other. Advantageously, the steerable device under control by a medical professional will also have suction functionality for performance of a particular medical function, while the inactive steerable device cannot inadvertently engage in suction. For example, a steerable portion fo the endoscope can also be used to suction any excess fluid or debris from the area being examined, thereby clearing the way for a clearer view of the organs or cavity via a camera.

In addition to switching steering and/or fluid functionality, the embodiments herein may additionally or alternatively be capable of switching other functionalities between various components. For example, the embodiments herein may be capable of switching between cameras (e.g., where each steerable device has a camera), electromechanical sensors, actuatable electronic devices (e.g., brushes or the like), or any other suitable device(s). This switchability may occur due to movement of the above-described selector rod's knob 175, where such movement engages an electronic switch that informs a computer or other electronic device of the selector rod's position, but other methodologies are also contemplated.

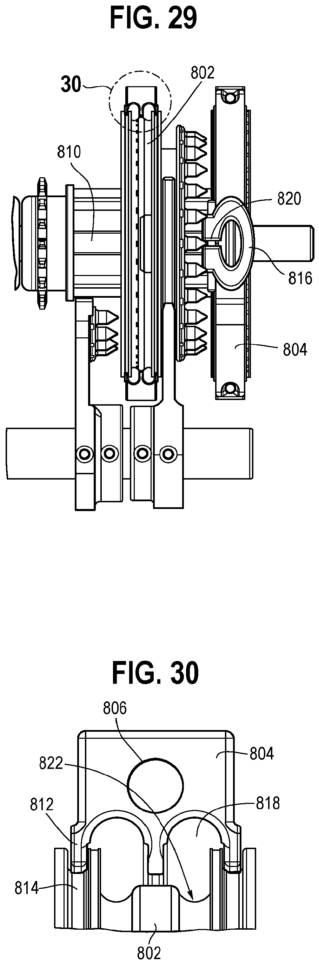

As illustrated by FIGS. 29-30 (and briefly mentioned above with reference to FIG. 26), one or more of the wire drum assemblies may include multiple components, such as a rotatable wire drum 802 (which may be a wheel, as discussed above) along with a drum harness 804 that at least partially surrounds the outer diameter surface of the rotatable wire drum 802. The wire drums 802 may be held with proper placement within the device via a drum harnesses, and the drum harness 804 may be substantially fixed with in the housing 130 (shown in FIG. 2) of the device, for example, via connection to the housing's shell using one or more attachment openings 806. E.g., the attachment openings 806 may accommodate a screw or other fastener type such that the drum harness 804 is installable within the housing in a fixed manner.

Referring to FIGS. 29-30, the drum harness 804 may generally function to maintain the position of the wire drum 802 within the housing of the device (e.g., in all three linear dimensions) while still allowing the wire drum 802 to rotate about the splineshaft 810 in accordance with the description above. In other words, the drum harness 804 may allow for rotation of a wire drum 802 while maintaining the wire drum 802 in a fixed linear position. Without the drum harness, the wire may unintentionally unspool from the drum in certain embodiments, and/or the wire may kink resulting in loss of steering or less responsive steering in certain embodiments. Accordingly, the drum harness 804 may include a protrusion 812, which may be ring-shaped and extend along the inner diameter of the drum harness 804, for the purposes of preventing axial-direction movement of the wire drum 802. The wire drum 802 may include a corresponding ring-shaped groove 814 for receiving the protrusion 812. Rotational movement of the wire drum 802 may cause the protrusion 812 to slide within the groove 814. Optionally, the surfaces of the groove 814 and/or the protrusion 812 may be configured for low friction, although a particular degree of friction may be desirable for additional feel-based control of the wire drum's rotation by a medical professional.

Optionally, two grooves 814 and/or two protrusions 812 may be included, one on each side of the wire drum 802, which may enhance the stability of the wire drum 802 within the drum harness 804. Additionally, or alternatively, the groove and protrusion may be swapped (e.g., the protrusion on the wire drum 802 and the groove on the drum harness 804), and/or different rotation-allowing components may be used in place of these features.

The drum harness 804 may include a wire exit opening 816, where the wire exit opening 816 provides access to a cavity 818 located between the wire drum 802 and the drum harness 804. When a steering wire (shown in FIG. ) is included, the steering wires may extend through the wire exit opening 816 such that it may wrap around a portion of the wire drum 802 and such that the wire drum's rotation will cause linear deflection of the steering wire. The exit opening 816 may be oval in shape, which creates sufficient room for two portions of the steering wire to extend therethrough, and also limits friction due to contact between the steering wire and the wire exit opening 816. Optionally, the wire exit opening 816 may be adjacent to an installation guide slot 820, which may provide installation access to the wire drum 802 when initially routing the guide wire, for example.

Without limitation, steering wires, when installed, may sit within the wire seats 822, which are generally formed as grooves having semi-circular cross-sections extending around the outer-diameter surface of the wire drums 802. Optionally, the steering wires may be encompassed, at least where engaging with the wire seats 822, within a sheath or tube, which may be advantageous for ensuring proper engagement between the wire drums 802 and the associated steering wires is achieved. Certain related features, advantages, and other aspects that may be utilized are described in U.S. Patent NO. 9,750,397, titled "MECHANISM OF SMALL DRIVE WIRE RETENTION ON SPOOL" and owned by Cook Medical Technologies LLC, which is hereby incorporated by reference in its entirety.

While various embodiments have been described, it will be apparent to those of ordinary skill in the art that many more embodiments and implementations are possible. Accordingly, the embodiments described herein are examples, not the only possible embodiments and implementations.

Having described various aspects of the subject matter above, additional disclosure is provided below that may be consistent with the claims originally filed with this disclosure. In describing this additional subject matter, reference may be made to the previously described figures. Any of the following aspects may be combined, where compatible.

One general aspect includes a medical scope device, a first steerable device extending to a distal end of the medical scope device. The medical scope device also includes and a second steerable device extending to the distal end of the medical scope device. The device also includes a first steering actuator. The device also includes and a selector switch that is mechanically coupled to the first steering actuator, where the first steering actuator controls a movement of a distal end of the first steerable device when the selector switch is in a first position, and where the first steering actuator controls a movement of a distal end of the second steerable device when the selector switch is in a second position.

Implementations may include one or more of the following features. The medical scope device may include a second steering actuator that controls a second movement of the distal end of the first steerable device when the selector switch is in the first position. The first steering actuator may be a first control wheel at a handle of the medical scope device, where the second steering actuator is a second control wheel at the handle, the first control wheel and the second control wheel being coaxial. The second steering actuator may control the second movement of the distal end of the second steerable device when the selector switch is in the second position. The medical scope device may include a first brake for selectively preventing rotation of the first steering actuator and a second brake for selectively preventing rotation of the second steering actuator. The first steering actuator may be rotationally fixed to a first spline shaft, and the second steering actuator is rotationally fixed to a second spline shaft. A first collar may be moveable along the first spline shaft linearly, where the first collar engages with a first wire drum when the selector switch is in the first position, and where the first collar engages with a second wire drum when the selector switch is in the second position. The first wire drum may be coupled to the first steerable device and where the second wire drum is coupled to the second steerable device. The collar may be supported via a collar mount, the collar mount being fixed relative to the selector switch, and the collar may be rotatable relative to the selector switch. The selector switch may be fixed relative to a lock ring, the lock ring being configured to engage at least one of the first wire drum and the second wire drum in a selected state to prevent rotation of said wire drum. The selector switch may be configured to selectively control fluid communication between two fluid ports of a fluid control system. Optionally, the at least one wire drum includes a wire harness that at least partially covers an outer diameter surface of the wire drum. When the selector switch is in the first position, a first port may be in fluid communication with a second port such that a fluid functionality is available at a distal end of the first steerable device, and where in the second position, the first port is fluidly isolated from the second port. When the selector switch is in the second position, the first port may be in fluid communication with a third port such that the fluid functionality is available at a distal end of the second steerable device. The second steerable device may be extendable distally relative to the first steerable device, where the first steering actuator selectively controls the second steerable device when the second steerable device is in an extended state. Optionally, the selector switch includes a rotatable knob for moving a selector rod in a linear direction to adjust the selector switch.

Another general aspect includes a medical scope device having a first steerable device extending to a distal end of the medical scope device. The medical scope device also includes a second steerable device extending to a distal end of the medical scope device. The device also includes a first control wheel. The device also includes a second control wheel. The device also includes and a selector switch having a rod that is coaxial with the first control wheel and the second control wheel, where the first control wheel controls a movement of the first steerable device when the selector switch is in a first position, and where the first control wheel controls a movement of a distal end of the second steerable device when the selector switch is in a second position.

Implementations may include one or more of the following features. The second steerable device may be moveable distally relative to the first steerable device, and the second steerable device may be controllable via the first control wheel when the second steerable device is in an extended state. At least one of the first wire drum and the second wire drum may move linearly when the rod of the selector switch moves linearly. The second wire drum may be configured to engage a countershaft gearwheel when the selector switch moves into a position for operating the second steerable device. The medical scope device may include a countershaft that is offset relative to, and parallel to, an axle that is coaxial with the first control wheel and the second control wheel. The collar may engage a countershaft gearwheel fo the countershaft such that rotation of the first control wheel causes rotation of the countershaft. The collar may include outer diameter teeth that selectively engage an inner diameter are of a first wire drum, where the first wire drum controls a movement of the first steerable device. The collar may include a shaft portion that aligns with the inner diameter of the first wire drum when the first wire drum is disengaged, and where the shaft portion is rotatable relative to the first wire drum when aligned with the inner diameter of the first wire drum. The first and second countershafts respectively may cause first and second motions of the first steerable device or the second steerable device based on the position of the selector switch.

Another general aspect includes a medical scope device with a steering system. The medical scope device also includes a first steerable device extending to a distal end of the medical scope device, where the steering system controls steering of the first steerable device when in a first state. The device also includes a second steerable device, where the steering system controls steering of the second steerable device when in a second state. The device also includes a selector rod configured to switch the steering system from the first state to the second state. The device also includes and a fluid control system, the fluid control system being mechanically coupled to the selector rod such that, in a first state, the fluid control system provides fluid communication between a first port and a second port. The device also includes and in a second state, the fluid control system prevents fluid communication between the first port and the second port.

Implementations may include one or more of the following features. The second port may include an interface for at least one of an insufflation flowpath, an irrigation flowpath, and a suction flowpath leading to a distal end of the first steerable device. A third port may be included, where the third port is in fluid communication with the first port when the fluid control system is in the second state. The third port may form fluid communication to at least one of an insufflation flowpath, an irrigation flowpath, and a suction flowpath leading to a distal end of the second steerable device. The fluid control system may include a fluid control housing may include at least one channel, where the selector rod is fixed to at least one fluid control rod that is linearly moveable within the at least one channel. The at least one channel may include a first channel and a second channel, where the at least one fluid control rod includes a first fluid control rod and a second fluid control rod that are linearly moveable within the respective first and second channels such that the fluid control system selectively controls at least two fluid functionalities.

## Claims

1. A medical scope device, comprising:
a first steerable device extending to a distal end of the medical scope device; and
a second steerable device extending to the distal end of the medical scope device;
a first steering actuator; and
a selector switch that is mechanically coupled to the first steering actuator,
wherein the first steering actuator controls a movement of a distal end of the first steerable device when the selector switch is in a first position, and
wherein the first steering actuator controls a movement of a distal end of the second steerable device when the selector switch is in a second position.

2. The medical scope device of claim 1, further comprising a second steering actuator that controls a second movement of the distal end of the first steerable device when the selector switch is in the first position.

3. The medical scope device of claim 2, wherein the first steering actuator is a first control wheel at a handle of the medical scope device, and wherein the second steering actuator is a second control wheel at the handle, the first control wheel and the second control wheel being coaxial.

4. The medical scope device of claim 2 or 3, wherein the second steering actuator controls the second movement of the distal end of the second steerable device when the selector switch is in the second position.

5. The medical scope device of any of claims 2 to 4, further comprising a first brake for selectively preventing rotation of the first steering actuator and a second brake for selectively preventing rotation of the second steering actuator.

6. The medical scope device of any preceding claim, wherein the first steering actuator is rotationally fixed to a first spline shaft.

7. The medical scope device of claim 6 as dependent upon claim 2, or as dependent on any of claims 3 to 5 as dependent upon claim 2, wherein the second steering actuator is rotationally fixed to a second spline shaft.

8. The medical scope device of claim 6 or 7, wherein a first collar is moveable along the first spline shaft linearly, wherein the first collar engages with a first wire drum when the selector switch is in the first position, and wherein the first collar engages with a second wire drum when the selector switch is in the second position.

9. The medical scope device of claim 8, wherein the first wire drum is coupled to the first steerable device and wherein the second wire drum is coupled to the second steerable device.

10. The medical scope device of claim 8 or 9, wherein the collar is supported via a collar mount, the collar mount being fixed relative to the selector switch, and wherein the collar is rotatable relative to the selector switch.

11. The medical scope device of any of claims 8 to 10, wherein the selector switch is fixed relative to a lock ring, the lock ring being configured to engage at least one of the first wire drum and the second wire drum in a selected state to prevent rotation of said wire drum.

12. The medical scope device of any of claims 8 to 11, wherein at least one wire drum includes a wire harness at least partially covering an outer diameter surface of the wire drum.

13. The medical scope device of any preceding claim, wherein the selector switch is configured to selectively control fluid communication between two fluid ports of a fluid control system, and wherein the selector switch optionally includes a rotatable knob for moving a selector rod in a linear direction to adjust the selector switch, for example, wherein when the selector switch is in the first position, a first port is in fluid communication with a second port such that a fluid functionality is available at a distal end of the first steerable device, and wherein in the second position, the first port is fluidly isolated from the second port.

14. The medical scope device of claim 13, wherein when the selector switch is in the second position, the first port is in fluid communication with a third port such that the fluid functionality is available at a distal end of the second steerable device.

15. The medical scope device of any preceding claim, wherein the second steerable device is extendable distally relative to the first steerable device, and wherein the first steering actuator selectively controls the second steerable device when the second steerable device is in an extended state.
